# EUROPEAN PATENT SPECIFICATION

(11) **EP 4 536 784 B1**
(45) Date of publication and mention of the grant of the patent: **17.06.2026**
(21) Application number: 23729131.5
(22) Date of filing: 30.05.2023
(51) Int. Cl.: C10M 135/10, C10L 10/18, C10L 1/24, C08F 8/02, C08F 8/34, C08F 110/10, C07C 303/06, C07C 309/31

(54) **POLYISOBUTYL BENZENE SULFONATE FOR LUBRICANTS AND FUELS**
POLYISOBUTYLBENZOLSULFONAT FÜR SCHMIERMITTEL UND KRAFTSTOFFE
SULFONATE DE POLYISOBUTYLBENZÈNE POUR LUBRIFIANTS ET CARBURANTS

(30) Priority: 10.06.2022 EP 22178339
(43) Date of publication of application: 16.04.2025
(73) Proprietor: BASF SE, 67056 Ludwigshafen am Rhein (DE)
(72) Inventor: PERETOLCHIN, Maxim, 67466 Lambrecht (DE); KOSCHABEK, Rene, 67056 Ludwigshafen am Rhein (DE); SCHUSTER, Thomas, 67056 Ludwigshafen am Rhein (DE)
(74) Representative: BASF IP Association
(86) International application number: PCT/EP2023/064428
(87) International publication number: WO 2023/237382

(56) References cited:
- EP-A1- 0 310 366
- US-A- 2 943 924
- US-A- 3 600 451
- US-A- 5 108 631

## Description

The present invention relates to a method for preparing a polyisobutyl benzene sulfonate comprising the steps of alkylation of benzene with a terminal unsaturated polyisobutene to produce a polyisobutyl benzene, sulfonation of the polyisobutyl benzene to produce a polyisobutyl benzene sulfonic acid, and neutralizing the polyisobutyl benzene sulfonic acid to produce a polyisobutyl benzene sulfonate. The invention also relates to the polyisobutyl benzene sulfonate, the polyisobutyl benzene sulfonic acid, the polyisobutyl benzene, and to a lubricant comprising the polyisobutyl benzene sulfonate, to a fuel comprising the polyisobutyl benzene sulfonate, and to a use of the polyisobutyl benzene sulfonate as lubricant additive or fuel additive. Combinations of preferred embodiments with other preferred embodiments are within the scope of the present invention.

Alkylbenzene sulfonates are useful additives for lubricants and fuels. EP 0 310 366 A1 and US 3 600 451 A disclose lubricating compositions comprising alkylbenzene sulfonates as additives, like polyisobutyl benzene sulfonates and their preparation.

Objects were to overcome disadvantages and to find an improved synthesis of alkylbenzene sulfonates and improved lubricants or fuels containing the alkylbenzene sulfonates. Further objective was to improve lubricants, where the lubricant has good rheological behavior, a high viscosity index, a good low temperature performance (e.g. in the cold crankcase simulation), a low viscosity under operating conditions (e.g. in the high temperature high shear HTHS viscosity test), a high shear stability, or a low viscosity loss after many use cycles. Further objects were to find fuel or lubricant additives with a higher solubility. Further objects were to find lubricant additives and fuel additives which result in an improved storage stability, prevent precipitations, corrosion protection, improve formulability (e.g. mixability with other components of the mixtures), or filterability.

The present invention is disclosed in and by the appended claims.

The objects were solved by a method for preparing a polyisobutyl benzene sulfonate comprising the steps of
i) alkylation of benzene with a terminal unsaturated polyisobutene to produce a polyisobutyl benzene,
ii) sulfonation of the polyisobutyl benzene to produce a polyisobutyl benzene sulfonic acid, and
iii) neutralizing the polyisobutyl benzene sulfonic acid to produce a polyisobutyl benzene sulfonate,
where the terminal unsaturated polyisobutene comprises at least 50 mol% of terminal double bonds, based on the total number of polyisobutene macromolecules.

The objects were also solved by the polyisobutyl benzene sulfonate. The objects were also solved by the polyisobutyl benzene sulfonic acid. The objects were also solved by the polyisobutyl benzene.

The polyisobutyl benzene is usually produced by **alkylation** of benzene with a terminal unsaturated polyisobutene.

The **terminal unsaturated polyisobutene** can be of the formula (b1), (b2), or mixtures thereof 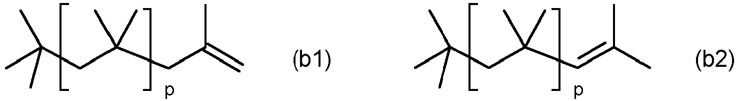 where p is in the range from 5 to 100, preferably from 8 to 80 and in particular in the range from 10 to 50. Preferred terminal unsaturated polyisobutene is of the formula (b1) where p is in the range from 5 to 100, preferably from 8 to 80 and in particular in the range from 10 to 50.

The terminal unsaturated polyisobutene can be a highly reactive polyisobutene. Highly reactive polyisobutenes are understood as meaning polyisobutenes with at least 50 mol%, often with at least 60 mol% and in particular with at least 80 mol%, based on the total number of polyisobutene macromolecules, of terminally arranged double bonds. The terminally arranged double bonds may either be vinyl double bonds [-CH=C(CH₃)₂] (□-olefin) or vinylidene double bonds [-CH-C(=CH₂)-CH_{3]} (□-olefin). Preferred highly reactive polyisobutenes have predominantly vinylidene double bonds, such as in formula (b1). Highly reactive polyisobutenes are commercially available, e.g. the Glissopal grades from BASF SE, thus e.g. Glissopal^{®} 1000 and Glissopal^{®} 1300, Glissopal^{®} 2300.

In another form the terminal unsaturated polyisobutene may comprise at least 50 mol%, preferably at least 60 mol% and in particular at least 80 mol%, based on the total number of polyisobutene macromolecules, of terminal double bonds.

The terminal unsaturated polyisobutene may be a technical product. The terminal unsaturated polyisobutene can comprise polymerized therein up to 20% by weight, preferably not more than 10% by weight, of C₂-C₁₂-olefins different from isobutene, such as 1-butene, 2-butene, 2-methyl-1-butene, 2-methylpentene-1, 2-methylhexene-1, 2-ethylpentene-1, 2-ethylhexene-1, 2-propylheptene-1.

The alkylation is usually a Friedel Crafts type reaction, in which typically an alkylation catalyst is used to catalyze an electrophilic aromatic substitution of an alkylating agent (e.g. an alkyl halide or alkene) to an aromatic ring.

Suitable **alkylation catalysts** for the alkylation are heterogeneous acids, hydrofluoric acid, aluminum chloride, or boron fluoride, where heterogeneous acids are preferred. Suitable alkylation catalysts for the alkylation are heterogeneous acids selected from
- clays, in particular montmorillonite or montmorillonite-containing materials, such as, for example, K10 and K20 from Südchemie,
- strongly acidic ion exchangers, such as Amberlyst^{®} 36 or Amberlyst^{®} 15 from Rohm & Haas or Nafion^{®} or Nafion^{®}/silica from DuPont,
- acidic metal oxides, such as, for example, M(I)O₃-M(II)O₂- M(I)=W, Mo and M(II) = Zr, Ti, Mn and/or Sn, Al₂O₃-SiO₂, TiO₂-ZrO₂, TiO₂, Nb₂O₅, Ta₂O₅,
- sulfated metal oxides, such as ZrO₂-SO₃, TiO₂-SO₃, Al₂O₃-SO₃, WO₃-SO₃, Nb₂O₅-SO₃,
- supported heteropolyacids, such as PW₁₂-HPA/SiO₂, PMo₁₂-HPA/coal, P₂W₁₈-HPA/TiO₂, and
- zeolites.

Preferred alkylation catalysts for the alkylation are zeolites.

Preference is given to zeolites of the structure types BIK; BRE, ERI, CHA, DAC, EAB, EDI, EPI, FER, pentasils with MFI or MEL structure, faujasites, such as, for example, Y, LTL, MOR, BEA, GME, HEU, KFI, MAZ, OFF, PAU, RHO, STI. Particular preference is given to L, Y including the USY types, BEA and MOR. These zeolites are preferably used in the H and/or La form, although traces of Na, K, Mg or Ca may be present depending on the preparation. Partial or complete exchange of the lattice aluminum by B, Ga or Fe is possible.

The catalyst can be used directly as fine powder in suspension, in the case of zeolites, these are, for example, particle sizes between 100 nm and a few µm. However, in most cases, these catalysts are shaped together with binder materials to give shaped bodies with a diameter of 0.1-5 mm. For use in fixed beds, 1-3 mm are preferred, in suspension 0.001-1 mm, in moving beds 0.1-3 mm. Suitable binders are in particular clays, aluminum oxides, such as, for example, Purals, Sirals and Versals and silica gels. In addition, inert fillers such as SiO₂ (e.g. Aerosil^{®} from Degussa) can be added. Examples of suitable shaped bodies are tablets, small strands, rings, ribbed strands, star or wheel extrudates.

The catalysts can have specific surface areas of from 30 to 2000 m²/g, preferably 100 to 700 m²/g. The volume of the pores with a diameter of 2-20 nm is typically 0.05-0.5 ml/g, preferably 0.1-0.3 ml/g, that of the pores of 20-200 nm is typically 0.005 to 0.2 ml/g, preferably 0.01 to 0.1 ml/g, and that of the pores of 200-2000 nm is typically 0.05-0.5 ml/g, preferably 0.05 to 0.3 ml/g.

Deactivated catalysts can in most cases be regenerated by burning off in air or depleted air at 250-550 °C. Alternatively, a treatment with compounds which have an oxidizing effect at lower temperatures-optionally also in the liquid phase-is possible, in this connection mention is made in particular of NOx, H₂O₂ and the halogens. The regeneration can take place directly in the alkylation reactor or externally.

The alkylation can take place in the liquid phase, i.e. without gas phase, which can be achieved by a corresponding system pressure. The pressure is usually the autogenous pressure (the vapor pressure of the system) or greater. Alkylation temperatures can be 100 to 250 °C, preferably 120 to 220 °C, in particular 130 to 200 °C. Suitable pressures are in the range from 1 to 35 bar.

The alkylation may be carried out in batch or continuous mode. In the batch mode, a typical method is to use a stirred autoclave or glass flask, which may be heated to the desired reaction temperature. A continuous process is most efficiently carried out in a fixed bed process. Space rates in a fixed bed process can range from 0.01 to 10 or more weight hourly space velocity. In a fixed bed process, the alkylation catalyst is charged to the reactor and activated or dried at a temperature of at least 150°C under vacuum or flowing inert, dry gas. After activation, the alkylation catalyst is cooled to ambient temperature and a flow of the aromatic hydrocarbon compound is introduced, optionally toluene. Pressure is increased by means of a back pressure valve so that the pressure is above the bubble point pressure of the aromatic hydrocarbon feed composition at the desired reaction temperature. After pressurizing the system to the desired pressure, the temperature is increased to the desired reaction temperature. A flow of the olefin is then mixed with the aromatic hydrocarbon and allowed to flow over the catalyst. The reactor effluent comprising alkylated aromatic hydrocarbon, unreacted olefin and excess aromatic hydrocarbon compound are collected. The excess aromatic hydrocarbon compound is then removed by distillation, stripping, evaporation under vacuum, or any other means known to those skilled in the art.

The polyisobutyl benzene sulfonic acid is usually produced by **sulfonation** of the polyisobutyl benzene.

The sulfonation the polyisobutyl benzene can be effected by reacting the polyisobutyl benzene with a sulfonation agent, such as with concentrated sulfuric acid, with an oleum, with sulfur trioxide dilute in nitrogen or air, or with sulfur trioxide dissolved in sulfur dioxide. The sulfonation with sulfur trioxide is preferred. The sulfonation reaction can be made in stirred vessels or in a falling film reactor.

After sulfonation the raw product can optionally be purified by conventional methods, such as washing with water or by thermal treatment with stirring by nitrogen bubbling.

The sulfonic acid group in the polyisobutyl benzene sulfonic acid can be in the ortho, meta, or para position to the polyisobutyl group, where the para position is preferred.

The sulfonate group in the polyisobutyl benzene sulfonate can be in the ortho, meta, or para position to the polyisobutyl group, where the para position is preferred.

The polyisobutyl benzene sulfonate is usually produced by **neutralizing** the polyisobutyl benzene sulfonic acid.

The polyisobutyl benzene sulfonic acid can be neutralized using a **base.** Examples of suitable bases include alkali (e.g. sodium, potassium), earth alkali (e.g. calcium, magnesium), ammonium or amine (e.g., isopropylamine, methylamine, triethanolamine) salts of hydroxide, carbonate, or oxides. For instance, sodium hydroxide, potassium hydroxide, ammonium hydroxide, sodium carbonate, sodium bicarbonate, potassium carbonate, magnesium hydroxide, magnesium carbonate, basic magnesium carbonate, calcium hydroxide, and calcium carbonate, and mixtures thereof may be used for neutralization.

Following neutralization the sulfonate may be purified, e.g. by filtration, centrifuging, washing, drying, and/or other methods for removing a solid sulfonate product from aqueous solution.

In a preferred form the method for preparing the polyisobutyl benzene sulfonate comprises the steps of
i) alkylation of benzene with a terminal unsaturated polyisobutene to produce a polyisobutyl benzene,
ii) sulfonation of the polyisobutyl benzene to produce a polyisobutyl benzene sulfonic acid, and
iii) neutralizing the polyisobutyl benzene sulfonic acid to produce a polyisobutyl benzene sulfonate,

where the alkylation catalyst for the alkylation is a zeolite,
where the sulfonation is effected by reacting the polyisobutyl benzene with sulfur trioxide, and
where for neutralizing a base is used selected from alkali, earth alkali, ammonium or amine salts of hydroxide, carbonate, or oxides.

In another preferred form the method for preparing the polyisobutyl benzene sulfonate comprises the steps of
i) alkylation of benzene with a terminal unsaturated polyisobutene to produce a polyisobutyl benzene,
ii) sulfonation of the polyisobutyl benzene to produce a polyisobutyl benzene sulfonic acid, and
iii) neutralizing the polyisobutyl benzene sulfonic acid to produce a polyisobutyl benzene sulfonate,

where the alkylation catalyst for the alkylation is a zeolite,
where the sulfonation is effected by reacting the polyisobutyl benzene with sulfur trioxide, and
where for neutralizing a base is used selected from alkali, earth alkali, ammonium or amine salts of hydroxide, carbonate, or oxides, and
where the terminal unsaturated polyisobutene is of the formula (b1), (b2), or mixtures thereof 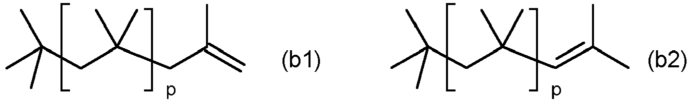
where p is in the range from 5 to 100, preferably from 8 to 80, and in particular from 10 to 50,

In another preferred form the method for preparing the polyisobutyl benzene sulfonate comprises the steps of
i) alkylation of benzene with a terminal unsaturated polyisobutene to produce a polyisobutyl benzene,
ii) sulfonation of the polyisobutyl benzene to produce a polyisobutyl benzene sulfonic acid, and
iii) neutralizing the polyisobutyl benzene sulfonic acid to produce a polyisobutyl benzene sulfonate,

where the alkylation catalyst for the alkylation is a zeolite,
where the sulfonation is effected by reacting the polyisobutyl benzene with sulfur trioxide, and
where for neutralizing a base is used selected from alkali, earth alkali, ammonium or amine salts of hydroxide, carbonate, or oxides, and
where the polyisobutyl group has a molecular weight of 300 to 10.000 g/mol, preferably from 400 to 5000 g/mol, and in particular from 400 to 3000 g/mol, and optionally where the sulfonic acid group is in para position to the polyisobutyl group.

The **polyisobutyl group** (e.g. in the polyisobutyl benzene sulfonate, the polyisobutyl benzene sulfonic acid, or the polyisobutyl benzene) can have a molecular weight of 300 to 10.000 g/mol, preferably from 400 to 5000 g/mol, and in particular from 400 to 3000 g/mol. The molecular weight can be determined by GPC using polystyrene standards, e.g. as described in DIN 55672-1.

The polyisobutyl is a monovalent radical of the formula (a) 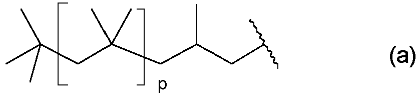 where p is in the range from 5 to 100, preferably from 8 to 80 and in particular in the range from 10 to 50.

The polyisobutyl group may be a technical product. The polyisobutyl group can comprise polymerized therein up to 20% by weight, preferably not more than 10% by weight, of C₂-C₁₂-olefins different from isobutene, such as 1-butene, 2-butene, 2-methyl-1-butene, 2-methylpentene-1, 2-methylhexene-1, 2-ethylpentene-1, 2-ethylhexene-1, 2-propylheptene-1. The polyisobutyl group can be derived from a highly reactive polyisobutene. Highly reactive polyisobutenes are understood as meaning polyisobutenes with at least 50 mol%, often with at least 60 mol% and in particular with at least 80 mol%, based on the total number of polyisobutene macromolecules, of terminally arranged double bonds. The terminally arranged double bonds may either be vinyl double bonds [-CH=C(CH₃)₂] (□-olefin) or vinylidene double bonds [-CH-C(=CH₂)-CH₃] (□-olefin). Preferred highly reactive polyisobutenes have predominantly vinylidene double bonds. Highly reactive polyisobutenes are commercially available, e.g. the Glissopal grades from BASF SE, thus e.g. Glissopal^{®} 1000 and Glissopal^{®} 1300, Glissopal^{®} 2300.

The **polyisobutyl benzene** is of the general structure (I) 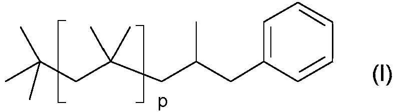 where p is in the range from 5 to 100, preferably from 8 to 80 and in particular in the range from 15 to 65.

The polyisobutyl benzene is preferably obtainable by a method comprising the step of
i) alkylation of benzene with the terminal unsaturated polyisobutene to produce the polyisobutyl benzene.

In another form the polyisobutyl benzene is obtainable by a method comprising the step of
i) alkylation of benzene with the terminal unsaturated polyisobutene to produce the polyisobutyl benzene,
where the polyisobutyl group has a molecular weight of 300 to 10.000 g/mol, preferably from 400 to 5000 g/mol, and in particular from 400 to 3000 g/mol.

In another form the polyisobutyl benzene is obtainable by a method comprising the step of
i) alkylation of benzene with the terminal unsaturated polyisobutene to produce the polyisobutyl benzene, where the alkylation catalyst for the alkylation is a zeolite, and

where the terminal unsaturated polyisobutene is of the formula (b1), (b2), or mixtures thereof 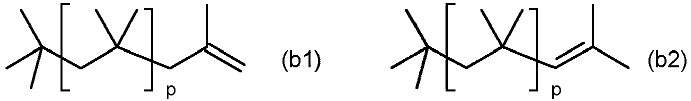
where p is in the range from 5 to 100, preferably from 8 to 80, and in particular from 10 to 50.

In another form the polyisobutyl benzene is obtainable by a method comprising the step of
i) alkylation of benzene with the terminal unsaturated polyisobutene to produce the polyisobutyl benzene, where the alkylation catalyst for the alkylation is a zeolite, and
where the polyisobutyl group has a molecular weight of 300 to 10.000 g/mol, preferably from 400 to 5000 g/mol, and in particular from 400 to 3000 g/mol.

Disclosed is also a method for preparing the polyisobutyl benzene comprising the step of alkylation of benzene with the terminal unsaturated polyisobutene to produce the polyisobutyl benzene.

The **polyisobutyl benzene sulfonic acid** is of the general structure (II) 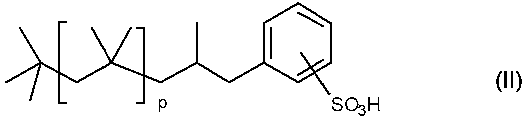 where p is in the range from 5 to 100, preferably from 8 to 80 and in particular in the range from 10 to 50, and the sulfonic acid group may be in ortho, meta or para position (where the para position is preferred).

The polyisobutyl benzene sulfonic acid is preferably obtainable by a method comprising the steps of
i) alkylation of benzene with the terminal unsaturated polyisobutene to produce the polyisobutyl benzene, and
ii) sulfonation of the polyisobutyl benzene to produce the polyisobutyl benzene sulfonic acid.

Disclosed is also a method for preparing the polyisobutyl benzene sulfonic acid comprising the steps of
i) alkylation of benzene with the terminal unsaturated polyisobutene to produce the polyisobutyl benzene, and
ii) sulfonation of the polyisobutyl benzene to produce the polyisobutyl benzene sulfonic acid.

In another form the method for preparing the polyisobutyl benzene sulfonic acid comprises the steps of
i) alkylation of benzene with the terminal unsaturated polyisobutene to produce the polyisobutyl benzene, and
ii) sulfonation of the polyisobutyl benzene to produce the polyisobutyl benzene sulfonic acid,

where the alkylation catalyst for the alkylation is a zeolite, and
where the terminal unsaturated polyisobutene is of the formula (b1), (b2), or mixtures thereof 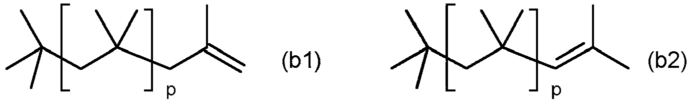
where p is in the range from 5 to 100, preferably from 8 to 80, and in particular from 10 to 50.

In another form the method for preparing the polyisobutyl benzene sulfonic acid comprises the steps of
i) alkylation of benzene with the terminal unsaturated polyisobutene to produce the polyisobutyl benzene, and
ii) sulfonation of the polyisobutyl benzene to produce the polyisobutyl benzene sulfonic acid,

where the alkylation catalyst for the alkylation is a zeolite, and
where the terminal unsaturated polyisobutene is of the formula (b1), (b2), or mixtures thereof 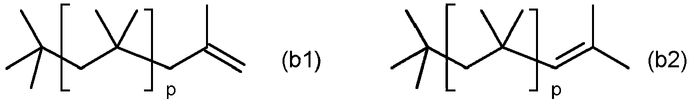
where p is in the range from 5 to 100, preferably from 8 to 80, and in particular from 10 to 50, where the sulfonation is effected by reacting the polyisobutyl benzene with sulfur trioxide, and
where the sulfonic acid group is in para position to the polyisobutyl group.

The **polyisobutyl benzene sulfonate** is of the general structure (III) 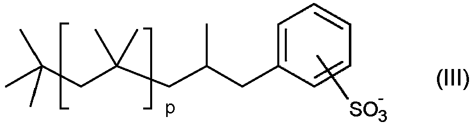 where p is in the range from 5 to 100, preferably from 8 to 80 and in particular in the range from 10 to 50, and the sulfonic acid group may be in ortho, meta or para position (where the para position is preferred).

The polyisobutyl benzene sulfonate is preferably obtainable by a method comprising the steps of
i) alkylation of benzene with the terminal unsaturated polyisobutene to produce the polyisobutyl benzene, and
ii) sulfonation of the polyisobutyl benzene to produce the polyisobutyl benzene sulfonic acid, and
iii) neutralizing the polyisobutyl benzene sulfonic acid to produce the polyisobutyl benzene sulfonate.

The polyisobutyl benzene sulfonate can have the form of a neutral or overbased alkali metal salt, earth alkali metal salt or ammonium salt.

The polyisobutyl benzene sulfonate can be present in form of a salt with a cationic counterion. The polyisobutyl benzene sulfonate can have the form of an alkali metal salt (e.g. lithium, sodium, potassium), earth alkali metal salt (e.g. magnesium, calcium, strontium, and barium), or ammonium salt.

In another form the polyisobutyl benzene sulfonate is a calcium salt of polyisobutyl benzene sulfonate, a magnesium salt of polyisobutyl benzene sulfonate, or a barium salt of polyisobutyl benzene sulfonate.

The polyisobutyl benzene sulfonate can be present as a **overbased sulfonate,** e.g. a low overbased sulfonate or a high overbased sulfonate.

Overbased sulfonates can be characterized by their base number (also termed BN). The BN refers to the amount of base equivalent to milligrams of KOH in one gram of sample. Thus, higher BN numbers reflect more alkaline products, and therefore a greater alkalinity reserve. The BN of a sample can be determined by ASTM Test No. D2896. A low overbased alkylaryl sulfonate often refers to an overbased sulfonate having a BN of about 2 to about 60. A high overbased alkylaryl sulfonate often refers to an overbased sulfonate having a BN of at least 250.

The overbased sulfonate can be prepared by adding a stoichiometric excess of the base (e.g. hydroxide, oxide, carbonate), based on the amount required to react with the acidic moiety of the sulfonate. Usually a diluent (e.g. a mineral oil) is incorporated in the overbased sulfonate.

The invention further relates to a **lubricant** comprising the polyisobutyl benzene sulfonate which is obtainable by a method for preparing the polyisobutyl benzene sulfonate comprising the steps of
i) alkylation of benzene with a terminal unsaturated polyisobutene to produce the polyisobutyl benzene,
ii) sulfonation of the polyisobutyl benzene to produce the polyisobutyl benzene sulfonic acid, and
iii) neutralizing the polyisobutyl benzene sulfonic acid to produce the polyisobutyl benzene sulfonate,
where the terminal unsaturated polyisobutene comprises at least 50 mol% of terminal double bonds, based on the total number of polyisobutene macromolecules.

In another form the lubricant comprises the polyisobutyl benzene sulfonate, where the polyisobutyl group has a molecular weight of 300 to 10.000 g/mol, preferably from 400 to 5000 g/mol, and in particular from 400 to 3000 g/mol.

According to the invention the lubricant comprises the polyisobutyl benzene sulfonate, where the polyisobutyl is a monovalent radical of the formula (a) 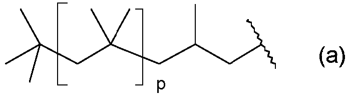 where p is in the range from 5 to 100, preferably from 8 to 80, and in particular from 10 to 50.

In another form the lubricant comprises the polyisobutyl benzene sulfonate, where the sulfonic acid group is in para position to the polyisobutyl group.

In another form the lubricant comprises the polyisobutyl benzene sulfonate, which has the form of a neutral or overbased alkali metal salt, earth alkali metal salt or ammonium salt.

Typically, the lubricant comprises
- a base oil,
- the polyisobutyl benzene sulfonate, and
- further lubricant additives.

The lubricant may comprise at least 0.1 wt%, preferably at least 0.5 wt% and in particular at least 1 wt% of the polyisobutyl benzene sulfonate. In another form the lubricant may comprise 0.1 - 20 wt%, preferably 0.1 - 15 wt% and in particular at least 0.1 - 10 wt% of the polyisobutyl benzene sulfonate.

The lubricant may comprise at least 30 wt%, preferably at least 50 wt% and in particular at least 70 wt% of the base oil. The lubricant may comprise 30 - 99.9 wt%, preferably 50 - 99 wt% and in particular 70 - 95 wt% of the base oil.

The lubricant may comprise up to 20 wt%, preferably up to 15 wt% and in particular up to 10 wt% of the further lubricant additive.

Lubricants usually refers to composition which are capable of reducing friction between surfaces (preferably metal surfaces), such as surfaces of mechanical devices. A mechanical device may be a mechanism consisting of a device that works on mechanical principles.

Suitable mechanical device are bearings, gears, joints and guidances. The mechanical device may be operated at temperatures in the range of -30 C to 80 °C.

The lubricant is usually a lubricating liquid, lubricating oil or lubricating grease.

Lubricants are usually specifically formulated for virtually every type of machine and manufacturing process. The type and concentration of base oils and/or lubricant additives used for these lubricants may be selected based on the requirements of the machinery or process being lubricated, the quality required by the builders and the users of the machinery, and the government regulation. Typically, each lubricant has a unique set of performance requirements. In addition to proper lubrication of the machine or process, these requirements may include maintenance of the quality of the lubricant itself, as well as the effect of the lubricant' s use and disposal on energy use, the quality of the environment, and on the health of the user.

Typical lubricants are automotive lubricants (e.g. gasoline engine oils, diesel engine oils, gas engine oils, gas turbine oils, automatic transmission fluids, gear oils) and industrial lubricants (e.g. industrial gear oils, pneumatic tool lubricating oil, high temperature oil, gas compressor oil, hydraulic fluids, metalworking fluids).

Examples for lubricants are axel lubrication, medium and heavy duty engine oils, industrial engine oils, marine engine oils, automotive engine oils, crankshaft oils, compressor oils, refrigerator oils, hydrocarbon compressor oils, very low-temperature lubricating oils and fats, high temperature lubricating oils and fats, wire rope lubricants, textile machine oils, refrigerator oils, aviation and aerospace lubricants, aviation turbine oils, transmission oils, gas turbine oils, spindle oils, spin oils, traction fluids, transmission oils, plastic transmission oils, passenger car transmission oils, truck transmission oils, industrial transmission oils, industrial gear oils, insulating oils, instrument oils, brake fluids, transmission liquids, shock absorber oils, heat distribution medium oils, transformer oils, fats, chain oils, minimum quantity lubricants for metalworking operations, oil to the warm and cold working, oil for water-based metalworking liquids, oil for neat oil metalworking fluids, oil for semi-synthetic metalworking fluids, oil for synthetic metalworking fluids, drilling detergents for the soil exploration, hydraulic oils, in biodegradable lubricants or lubricating greases or waxes, chain saw oils, release agents, molding fluids, gun, pistol and rifle lubricants or watch lubricants and food grade approved lubricants.

The lubricant has usually may have a kinematic viscosity at 40 °CC of at least 10, 50, 100, 150, 200, 300, 400, 500, 600, 900, 1400, or 2000 mm²/s. In another form the lubricant has usually may have a kinematic viscosity at 40 °C from 200 to 30 000 mm²/s (cSt), preferably from 500 to 10 000 mm²/s, and in particular from 1000 to 5000 mm²/s.

The lubricant has usually may have a kinematic viscosity at 100 °C of at least 2, 3, 5, 10, 20, 30, 40, or 50 mm²/s. In another form the lubricant may have a kinematic viscosity at 100 °C from 10 to 5000 mm²/s (cSt), preferably from 30 to 3000 mm²/s, and in particular from 50 to 2000 mm²/s

The lubricant may have a viscosity index (VI) of at least 150, 160, 170, 180, 190 or 200.

The **base oil** may selected from the group consisting of mineral oils (Group I, II or III oils), polyalphaolefins (Group IV oils), polymerized and interpolymerized olefins, alkyl naphthalenes, alkylene oxide polymers, silicone oils, phosphate esters and carboxylic acid esters (Group V oils). Preferably, the base oil is selected from Group I, Group II, Group III base oils according to the definition of the API, or mixtures thereof. Definitions for the base oils are the same as those found in the American Petroleum Institute (API) publication "Engine Oil Licensing and Certification System", Industry Services Department, Fourteenth Edition, December 1996, Addendum 1, December 1998. Said publication categorizes base oils as follows:
a) Group I base oils contain less than 90 percent saturates (ASTM D 2007) and/or greater than 0.03 percent sulfur (ASTM D 2622) and have a viscosity index (ASTM D 2270) greater than or equal to 80 and less than 120.
b) Group II base oils contain greater than or equal to 90 percent saturates and less than or equal to 0.03 percent sulfur and have a viscosity index greater than or equal to 80 and less than 120.
c) Group III base oils contain greater than or equal to 90 percent saturates and less than or equal to 0.03 percent sulfur and have a viscosity index greater than or equal to 120.
d) Group IV base oils contain polyalphaolefins. Polyalphaolefins (PAO) include known PAO materials which typically comprise relatively low molecular weight hydrogenated polymers or oligomers of alphaolefins which include but are not limited to C2 to about C32 alphaolefins with the C8 to about C16 alphaolefins, such as 1-octene, 1-decene, 1-dodecene and the like being preferred. The preferred polyalphaolefins are poly-1-octene, poly-1-decene, and poly-1-dode-cene.
e) Group V base oils contain any base oils not described by Groups I to IV. Examples of Group V base oils include alkyl naphthalenes, alkylene oxide polymers, silicone oils, and phosphate esters.

Synthetic base oils include hydrocarbon oils and halo-substituted hydrocarbon oils such as polymerized and interpolymerized olefins (e.g., polypropylenes, propylene-isobutylene copolymers, chlorinated polybutylenes, poly(1-hexenes), poly(1-octenes), poly(1-decenes)); alkylbenzenes (e.g., dodecylbenzenes, tetradecylbenzenes, dinonylbenzenes, di(2-ethylhexyl)benzenes); poly-phenyls (e.g., biphenyls, terphenyls, alkylated polyphenols); and alkylated diphenyl ethers and alkylated diphenyl sulfides and derivative, analogs and homologs thereof.

Alkylene oxide polymers and interpolymers and derivatives thereof where the terminal hydroxyl groups have been modified by esterification, etherification, etc., constitute another class of known synthetic base oils. These are exemplified by polyoxyalkylene polymers prepared by polymeriza-tion of ethylene oxide or propylene oxide, and the alkyl and aryl ethers of polyoxyalkylene poly-mers (e.g., methyl-polyisopropylene glycol ether having a molecular weight of 1000 or diphenyl ether of polyethylene glycol having a molecular weight of 1000 to 1500); and mono- and polycar-boxylic esters thereof, for example, the acetic acid esters, mixed C3-C8 fatty acid esters and C13 oxo acid diester of tetraethylene glycol.

Silicon-based oils such as the polyalkyl-, polyaryl-, polyalkoxy- or polyaryloxysilicone oils and sili-cate oils comprise another useful class of synthetic base oils; such base oils include tetraethyl silicate, tetraisopropyl silicate, tetra-(2- ethylhexyl)silicate, tetra-(4-methyl-2-ethylhe-xyl) silicate, tetra-(p-tert-butyl-phenyl) silicate, hexa-(4-methyl-2-ethylhexyl)disiloxane, poly(methyl) siloxanes and poly(methylphenyl)siloxanes. Other synthetic base oils include liquid esters of phosphorous-containing acids (e.g., tricresyl phosphate, trioctyl phosphate, diethyl ester of decylphosphonic acid) and polymeric tetrahydrofurans.

The lubricant may further comprise a **lubricant additive.** Suitable lubricant additives may be selected from viscosity index improvers, polymeric thickeners, corrosion inhibitors, detergents, dispersants, anti-foam agents, dyes, wear protection additives, extreme pressure additives (EP additives), anti-wear additives (AW additives), friction modifiers, metal deactivators, pour point depressants.

The total combined amount of the lubricant additive in the lubricant may include ranges of 0-25 wt%, or 0.01-20 wt%, or 0.1-15 wt% or 0.5-10 wt%, or 1-5 wt% of the lubricant.

The **viscosity index improvers** include high molecular weight polymers that increase the relative viscosity of an oil at high temperatures more than they do at low temperatures. Viscosity index improvers include polyacrylates, polymethacrylates, alkylmethacrylates, vinylpyrrolidone/meth-acrylate copolymers, poly vinylpyrrolidones, polybutenes, olefin copolymers such as an ethylene-propylene copolymer or a styrene-butadiene copolymer or polyalkene such as PIB, styrene/acrylate copolymers and polyethers, and combinations thereof. The most common VI improvers are methacrylate polymers and copolymers, acrylate polymers, olefin polymers and copolymers, and styrenebutadiene copolymers. Other examples of the viscosity index improver include polymethacrylate, polyisobutylene, alpha-olefin polymers, alpha-olefin copolymers (e.g., an ethylenepropylene copolymer), polyalkylstyrene, phenol condensates, naphthalene condensates, a styrenebutadiene copolymer and the like. Of these, polymethacrylate having a number average molecular weight of 10000 to 300000, and alpha-olefin polymers or alpha-olefin copolymers having a number average molecular weight of 1000 to 30000, particularly ethylene- alpha-olefin copolymers having a number average molecular weight of 1000 to 10000 are preferred. The viscosity index increasing agents can be added and used individually or in the form of mixtures, conveniently in an amount within the range of from ≥ 0.05 to ≤ 20.0 % by weight, in relation to the weight of the base stock.

Suitable (polymeric) **thickeners** include, but are not limited to, polyisobutenes (PIB), oligomeric co-polymers (OCPs), polymethacrylates (PMAs), copolymers of styrene and butadiene, or high viscosity esters (complex esters).

**Corrosion inhibitors** may include various oxygen-, nitrogen-, sulfur-, and phosphorus-containing materials, and may include metal-containing compounds (salts, organometallics, etc.) and nonmetal-containing or ashless materials. Corrosion inhibitors may include, but are not limited to, additive types such as, for example, hydrocarbyl-, aryl-, alkyl-, arylalkyl-, and alkylaryl versions of detergents (neutral, overbased), sulfonates, phenates, salicylates, alcoholates, carboxylates, salixarates, phosphites, phosphates, thiophosphates, amines, amine salts, amine phosphoric acid salts, amine sulfonic acid salts, alkoxylated amines, etheramines, polyetheramines, amides, imides, azoles, diazoles, triazoles, benzotriazoles, benzothiadoles, mercaptobenzothiazoles, tolyltriazoles (TTZ-type), heterocyclic amines, heterocyclic sulfides, thiazoles, thiadiazoles, mercaptothiadiazoles, dimercaptothiadiazoles (DMTD-type), imidazoles, benzimidazoles, dithiobenzimidazoles, imidazolines, oxazolines, Mannich reactions products, glycidyl ethers, anhydrides, carbamates, thiocarbamates, dithiocarbamates, polyglycols, etc., or mixtures thereof.

**Detergents** include cleaning agents that adhere to dirt particles, preventing them from attaching to critical surfaces. Detergents may also adhere to the metal surface itself to keep it clean and prevent corrosion from occurring. Detergents include calcium alkylsalicylates, calcium alkylphenates and calcium alkarylsulfonates with alternate metal ions used such as magnesium, barium, or sodium. Examples of the cleaning and dispersing agents which can be used include metal-based detergents such as the neutral and basic alkaline earth metal sulphonates, alkaline earth metal phenates and alkaline earth metal salicylates alkenylsuccinimide and alkenylsuccinimide esters and their borohydrides, phenates, salienius complex detergents and ashless dispersing agents which have been modified with sulphur compounds. These agents can be added and used individually or in the form of mixtures, conveniently in an amount within the range of from ≥ 0.01 to ≤ 1.0 % by weight in relation to the weight of the base stock; these can also be high total base number (TBN), low TBN, or mixtures of high/low TBN.

Dispersants are lubricant additives that help to prevent sludge, varnish and other deposits from forming on critical surfaces. The dispersant may be a succinimide dispersant (for example N-substituted long chain alkenyl succinimides), a Mannich dispersant, an ester-containing dispersant, a condensation product of a fatty hydrocarbyl monocarboxylic acylating agent with an amine or ammonia, an alkyl amino phenol dispersant, a hydrocarbyl-amine dispersant, a polyether dispersant or a polyetheramine dispersant. In one embodiment, the succinimide dispersant includes a polyisobutylene-substituted succinimide, wherein the polyisobutylene from which the dispersant is derived may have a number average molecular weight of about 400 to about 5000, or of about 950 to about 1600. In one embodiment, the dispersant includes a borated dispersant. Typically, the borated dispersant includes a succinimide dispersant including a polyisobutylene succinimide, wherein the polyisobutylene from which the dispersant is derived may have a number average molecular weight of about 400 to about 5000. Borated dispersants are described in more detail above within the extreme pressure agent description.

**Anti-foam agents** may be selected from silicones, polyacrylates, and the like. The amount of anti-foam agent in the lubricant compositions described herein may range from ≥ 0.001 wt.-% to≤ 0.1 wt.-% based on the total weight of the formulation. As a further example, an anti-foam agent may be present in an amount from about 0.004 wt.-% to about 0.008 wt.-%.

Suitable **extreme pressure agent** is a sulfurcontaining compound. In one embodiment, the sulfur-containing compound may be a sulfurised olefin, a polysulfide, or mixtures thereof. Examples of the sulfurised olefin include a sulfurised olefin derived from propylene, isobutylene, pentene; an organic sulfide and/or polysulfide including benzyldisulfide; bis-(chlorobenzyl) disulfide; dibutyl tetrasulfide; di-tertiary butyl polysulfide; and sulfurised methyl ester of oleic acid, a sulfurised alkylphenol, a sulfurised dipentene, a sulfurised terpene, a sulfurised Diels-Alder adduct, an alkyl sulphenyl N'N dialkyl dithiocarbamates; or mixtures thereof. In one embodiment, the sulfurised olefin includes a sulfurised olefin derived from propylene, isobutylene, pentene or mixtures thereof. In one embodiment the extreme pressure additive sulfur-containing compound includes a dimercaptothiadiazole or derivative, or mixtures thereof. Examples of the dimercaptothiadiazole include compounds such as 2,5-dimercapto-1,3,4-thiadiazole or a hydrocarbyl-substituted 2,5-dimercapto-1,3,4-thiadiazole, or oligomers thereof. The oligomers of hydrocarbyl-substituted 2,5-dimercapto-1,3,4-thiadiazole typically form by forming a sulfur-sulfur bond between 2,5-dimercapto-1,3,4-thiadiazole units to form derivatives or oligomers of two or more of said thiadiazole units. Suitable 2,5-dimercapto-1,3,4-thiadiazole derived compounds include for example 2,5-bis(tert-nonyldithio)-1,3,4-thiadiazole or 2-tert-nonyldithio-5-mercapto-1,3,4-thiadiazole. The number of carbon atoms on the hydrocarbyl substituents of the hydrocarbyl-substituted 2,5-dimercapto-1,3,4-thiadiazole typically include 1 to 30, or 2 to 20, or 3 to 16. Extreme pressure additives include compounds containing boron and/or sulfur and/or phosphorus. The extreme pressure agent may be present in the lubricant compositions at 0 wt.-% to about 20 wt.-%, or at about 0.05 wt.-% to about 10.0 wt.-%, or at about 0.1 wt.-% to about 8 wt.-% of the lubricant composition.

Examples of **anti-wear additives** include organo borates, organo phosphites such as didodecyl phosphite, organic sulfur-containing compounds such as sulfurized sperm oil or sulfurized terpenes, zinc dialkyl dithiophosphates, zinc diaryl dithiophosphates, phosphosulfurized hydrocarbons and any combinations thereof.

**Friction modifiers** may include metal-containing compounds or materials as well as ashless compounds or materials, or mixtures thereof. Metal-containing friction modifiers include metal salts or metalligand complexes where the metals may include alkali, alkaline earth, or transition group metals. Such metal-containing friction modifiers may also have lowash characteristics. Transition metals may include Mo, Sb, Sn, Fe, Cu, Zn, and others. Ligands may include hydrocarbyl derivative of alcohols, polyols, glycerols, partial ester glycerols, thiols, carboxylates, carbamates, thiocarbamates, dithiocarbamates, phosphates, thiophosphates, dithiophosphates, amides, imides, amines, thiazoles, thiadiazoles, dithiazoles, diazoles, triazoles, and other polar molecular functional groups containing effective amounts of O, N, S, or P, individually or in combination. In particular, Mo-containing compounds can be particularly effective such as for example Mo-dithiocarbamates, Mo(DTC), Mo-dithiophosphates, Mo(DTP), Mo-amines, Mo (Am), Mo-alcoholates, Mo alcohol-amides, and the like.

Ashless friction modifiers may also include lubricant materials that contain effective amounts of polar groups, for example, hydroxyl-containing hydrocarbyl base oils, glycerides, partial glycerides, glyceride derivatives, and the like. Polar groups in friction modifiers may include hydrocarbyl groups containing effective amounts of O, N, S, or P, individually or in combination. Other friction modifiers that may be particularly effective include, for example, salts (both ash-containing and ashless derivatives) of fatty acids, fatty alcohols, fatty amides, fatty esters, hydroxyl-containing carboxylates, and comparable synthetic long-chain hydrocarbyl acids, alcohols, amides, esters, hydroxy carboxylates, and the like. In some instances, fatty organic acids, fatty amines, and sulfurized fatty acids may be used as suitable friction modifiers. Examples of friction modifiers include fatty acid esters and amides, organo molybdenum compounds, molybdenum dialkylthiocarbamates and molybdenum dialkyl dithiophosphates.

Suitable **metal deactivators** include benzotriazoles and derivatives thereof, for example 4- or 5-alkylbenzotriazoles (e.g. triazole) and derivatives thereof, 4,5,6,7-tetrahydrobenzotriazole and 5,5'-methylenebisbenzotriazole; Mannich bases of benzotriazole or triazole, e.g. 1-[bis(2-ethyl-hexyl) aminomethyl) triazole and 1-[bis(2- ethylhexyl) aminomethyl)benzotriazole; and alkoxy-alkylbenzotriazoles such as 1-(nonyloxymethyl)benzotriazole, 1-(1-butoxyethyl) benzotriazole and 1-(1-cyclohexyloxybutyl) triazole, and combinations thereof. Additional non-limiting examples of the one or more metal deactivators include 1,2,4-triazoles and derivatives thereof, for example 3-alkyl(or aryl)-1, 2,4-triazoles, and Mannich bases of 1,2,4-triazoles, such as 1-[bis(2-ethylhexyl) aminomethy1-1, 2,4-triazole; alkoxyalky1-1, 2,4-triazoles such as 1-(1-bu-toxyethyl)-1, 2,4-triazole; and acylated 3-amino-1, 2,4-triazoles, imidazole derivatives, for example 4,4'-methylenebis(2-undecyl-5-methylimidazole) and bis[(N-methyl)imidazol-2-yl]-carbinol octyl ether, and combinations thereof. Further non-limiting examples of the one or more metal deactivators include sulfur-containing heterocyclic compounds, for example 2-mercaptobenzothiazole, 2,5-dimercapto-1, 3,4-thia-diazole and derivatives thereof; and 3,5-bis[di(2-ethylhexyl) aminomethyl]-1, 3,4-thiadiazolin-2-one, and combinations thereof. Even further non-limiting examples of the one or more metal deactivators include amino compounds, for example salicylidenepropylenediamine, salicylami-noguanidine and salts thereof, and combinations thereof. The one or more metal deactivators are not particularly limited in amount in the composition but are typically present in an amount of from about 0.01 to about 0.1, from about 0.05 to about 0.01, or from about 0.07 to about 0.1, wt.-% based on the weight of the composition. Alternatively, the one or more metal deactivators may be present in amounts of less than about 0.1, of less than about 0.7, or less than about 0.5, wt.-% based on the weight of the composition.

**Pour point depressants** (PPD) include polymethacrylates, alkylated naphthalene derivatives, and combinations thereof. Commonly used additives such as alkylaromatic polymers and polymethacrylates are also useful for this purpose. Typically, the treat rates range from ≥ 0.001 wt.-% to ≤ 1.0 wt.-%, in relation to the weight of the base stock. **Demulsifiers** include trialkyl phosphates, and various polymers and copolymers of ethylene glycol, ethylene oxide, propylene oxide, or mixtures thereof.

The invention also relates to a **fuel** comprising the polyisobutyl benzene sulfonate which is obtainable by a method for preparing the polyisobutyl benzene sulfonate comprising the steps of
i) alkylation of benzene with a terminal unsaturated polyisobutene to produce the polyisobutyl benzene,
ii) sulfonation of the polyisobutyl benzene to produce the polyisobutyl benzene sulfonic acid, and
iii) neutralizing the polyisobutyl benzene sulfonic acid to produce the polyisobutyl benzene sulfonate,
where the terminal unsaturated polyisobutene comprises at least 50 mol% of terminal double bonds, based on the total number of polyisobutene macromolecules.

In another form the fuel comprises the polyisobutyl benzene sulfonate, where the polyisobutyl group has a molecular weight of 300 to 10.000 g/mol, preferably from 400 to 5000 g/mol, and in particular from 400 to 3000 g/mol.

According to the invention the fuel comprises the polyisobutyl benzene sulfonate, where the polyisobutyl is a monovalent radical of the formula (a) 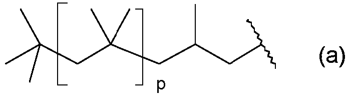 where p is in the range from 5 to 100, preferably from 8 to 80, and in particular from 10 to 50.

In another form the fuel comprises the polyisobutyl benzene sulfonate, where the sulfonic acid group is in para position to the polyisobutyl group.

In another form the fuel comprises the polyisobutyl benzene sulfonate, which has the form of a neutral or overbased alkali metal salt, earth alkali metal salt or ammoium salt.

The fuel may comprise at least 1 mg/kg, preferably at least 10 mg/kg and in particular at least 50 mg/kg of the polyisobutyl benzene sulfonate.

In another form the fuel (such as automotive fuels for cars or trucks) may comprise 1 mg/kg - 1000 mg/kg, preferably 10 mg/kg - 500 mg/kg and in particular at least 50 - 200 mg/kg of the polyisobutyl benzene sulfonate.

In another form (such as for marine fuel or aviation fuel) the fuel may comprise 0.001 - 10 wt%, preferably 0.001 - 5 wt% and in particular at least 0.01 - 2.5 wt% of the polyisobutyl benzene sulfonate.

The fuel can be understood to mean middle distillate fuels of fossil, vegetable or animal origin, biofuel ("biodiesel") and mixtures of such middle distillate fuels and biofuels.

**Middle distillate fuels** (also called "middle distillates" for short hereinafter) are especially understood to mean fuels which are obtained by distilling crude oil as the first process step and boil within the range from 120 to 450°C. Such middle distillate fuels are used especially as diesel fuel, heating oil or kerosene, particular preference being given to diesel fuel and heating oil. Preference is given to using low-sulfur middle distillates, i.e. those which comprise less than 350 ppm of sulfur, especially less than 200 ppm of sulfur, in particular less than 50 ppm of sulfur. In special cases they comprise less than 10 ppm of sulfur; these middle distillates are also referred to as "sulfur-free". They are generally crude oil distillates which have been subjected to refining under hydrogenating conditions and therefore comprise only small proportions of polyaromatic and polar compounds. They are preferably those middle distillates which have 90% distillation points below 370°C, especially below 360°C and in special cases below 330°C.

Low-sulfur and sulfur-free middle distillates may also be obtained from relatively heavy mineral oil fractions which cannot be distilled under atmospheric pressure. Typical conversion processes for preparing middle distillates from heavy crude oil fractions include: hydrocracking, thermal cracking, catalytic cracking, coking processes and/or visbreaking.

Depending on the process, these middle distillates are obtained in low-sulfur or sulfur-free form, or are subjected to refining under hydrogenating conditions. The middle distillates preferably have aromatics contents of below 28% by weight, especially below 20% by weight. The content of normal paraffins is between 5% by weight and 50% by weight, preferably between 10 and 35% by weight.

In the context of the present invention, middle distillate fuels shall also be understood here to mean those fuels which can either be derived indirectly from fossil sources such as mineral oil or natural gas, or else are produced from biomass via gasification and subsequent hydrogenation. A typical example of a middle distillate fuel which is derived indirectly from fossil sources is the GTL ("gas-to-liquid") diesel fuel obtained by means of Fischer-Tropsch synthesis. A middle distillate is prepared from biomass, for example, via the BTL ("biomass-to-liquid") process, and can be used as fuel either alone or in a mixture with other middle distillates. The middle distillates also include hydrocarbons which are obtained by the hydrogenation of fats and fatty oils. They comprise predominantly n-paraffins.

In a preferred embodiment the fuel is a diesel fuel (absent any additives) with a CP value according to ASTM D2500/ASTM D97 of 0 to -15 °C, preferably 0 to -10 °C, and more preferably -5 to -10 °C and/or, preferably and with a content of n-paraffines of from 10 to 27 % by weight, more preferably of from 15 to 25 % by weight, and most preferably from 17 to 23 % by weight.

In addition to its use in the middle distillate fuels of fossil, vegetable or animal origin mentioned, which are essentially hydrocarbon mixtures, the inventive copolymer can also be used in biofuel oils and in mixtures of the middle distillates mentioned with biofuel oils, in order to improve cold flow characteristics. Mixtures of this kind are commercially available and usually comprise the biofuel oils in minor amounts, typically in amounts of 1% to 30% by weight, especially of 3% to 10% by weight, based on the total amount of middle distillate of fossil, vegetable or animal origin and biofuel oil.

**Biofuel** are generally based on fatty acid esters, preferably essentially on alkyl esters of fatty acids which derive from vegetable and/or animal oils and/or fats. Alkyl esters are preferably understood to mean lower alkyl esters, especially C₁- to C₄-alkyl esters, which are obtainable by transesterifying the glycerides which occur in vegetable and/or animal oils and/or fats, especially triglycerides, by means of lower alcohols, for example ethanol or in particular methanol ("FAME"). Typical lower alkyl esters which are based on vegetable and/or animal oils and/or fats and find use as a biofuel oil or components thereof are, for example, HVO (hydrogenated vegetable oil), sunflower methyl ester, palm oil methyl ester ("PME"), soya oil methyl ester ("SME") and especially rapeseed oil methyl ester ("RME").

The fuel may be a **marine fuel,** such as MGO (Marine gas oil), MDO (Marine diesel oil), IFO (Intermediate fuel oil), MFO (Marine fuel oil), or HFO (Heavy fuel oil). Further examples for marine fuel are IFO 380 (an Intermediate fuel oil with a maximum viscosity of 380 centistokes (<3.5% sulphur)), IFO 180 (an Intermediate fuel oil with a maximum viscosity of 180 centistokes (<3.5% sulphur)), LS 380 (a Low-sulphur (<1.0%) intermediate fuel oil with a maximum viscosity of 380 centistokes), LS 180 (a Low-sulphur (<1.0%) intermediate fuel oil with a maximum viscosity of 180 centistokes), LSMGO (a Low-sulphur (<0.1%) Marine Gas Oil, which is to often be used in European Ports and Anchorages according to EU Sulphur directive 2005/33/EC), or ULSMGO (a Ultra-Low-Sulphur Marine Gas Oil, also referred to as Ultra-Low-Sulfur Diesel (sulphur 0.0015% max). Further suitable marine fuels are according to DIN ISO 8217 of the category ISO-F- DMX, DMA, DFA, DMZ, DFZ, or DFB, or ISO-F RMA, RMB, RMD, RME, RMG, or RMK. Further suitable marine fuel is distillate marine diesel or residual marine diesel.

The fuel may comprise further additives, such as carrier oils, cold flow improvers, lubricity improvers, corrosion inhibitors, dehazers, antifoams, cetane number improvers, combustion improvers, antioxidants or stabilizers, antistats, metallocenes, metal deactivators, and/or dyes.

The invention also relates to a use of the polyisobutyl benzene sulfonate as defined in the appended claims, as lubricant additive or fuel additive. The polyisobutyl benzene sulfonate can be added to the lubricants or the fuels, e.g. for improving their performance.

The lubricant additive can comprise 1 - 80 wt%, preferably 5 - 50 wt% and in particular at least 20 - 70 wt% of the polyisobutyl benzene sulfonate. The lubricant additive can comprise further lubricant additives or a base oil, e.g. a mineral oil.

The fuel additive can comprise 1 - 80 wt%, preferably 5 - 50 wt% and in particular at least 20 - 70 wt% of the polyisobutyl benzene sulfonate. The fuel additive can comprise further lubricant additives or a base oil, e.g. a mineral oil.

### Examples

### Example 1 -Polyisobutyl benzene with polyisobutyl group Mn 1000 g/mol

A polyisobutyl benzene is prepared starting from benzene and a terminal unsaturated polyisobutene with Mn of 1000 g/mol (as determined by GPC), an alpha olefin content of at least 85 % (as determined by GC), a bromine number of about 17 (ASTM D 1159) and pour point of -9 °C (ISO 3016), commercially available from BASF SE as Glissopal^{®} 1000. The alkylation catalyst is a zeolite and the Friedel-Crafts alkylation is successfully made to produce the corresponding polyisobutyl benzene.

### Example 2 -Polyisobutyl benzene with polyisobutyl group Mn 1300 g/mol

A polyisobutyl benzene is prepared starting from benzene and a terminal unsaturated polyisobutene with Mn of 1300 g/mol (as determined by GPC), an alpha olefin content of at least 85 % (as determined by GC), a bromine number of about 13 (ASTM D 1159) and pour point of -6 °C (ISO 3016), commercially available from BASF SE as Glissopal^{®} 1300. The alkylation catalyst is a zeolite and the Friedel-Crafts alkylation is successfully made to produce the corresponding polyisobutyl benzene.

### Example 3 -Polyisobutyl benzene with polyisobutyl group Mn 2000 g/mol

A polyisobutyl benzene is prepared starting from benzene and a terminal unsaturated polyisobutene with Mn of 2000 g/mol (as determined by GPC), an alpha olefin content of at least 85 % (as determined by GC), a bromine number of about 7.5 (ASTM D 1159) and pour point of 0 °C (ISO 3016), commercially available from BASF SE as Glissopal^{®} 2000. The alkylation catalyst is a zeolite and the Friedel-Crafts alkylation is successfully made to produce the corresponding polyisobutyl benzene.

### Example 4 -Polyisobutyl benzene sulfonic acid with polyisobutyl group Mn 1000 g/mol

A polyisobutyl benzene sulfonic acid is prepared by sulfonation of the polyisobutyl benzene from Example 1. The sulfonation is effected by reacting the polyisobutyl benzene with sulfur trioxide. The sulfonic acid group is mainly in para position to the polyisobutyl group, which can be determined by H-NMR.

### Example 5 -Polyisobutyl benzene sulfonic acid with polyisobutyl group Mn 1300 g/mol

A polyisobutyl benzene sulfonic acid is prepared by sulfonation of the polyisobutyl benzene from Example 2. The sulfonation is effected by reacting the polyisobutyl benzene with sulfur trioxide. The sulfonic acid group is mainly in para position to the polyisobutyl group, which can be determined by H-NMR.

### Example 6 -Polyisobutyl benzene sulfonic acid with polyisobutyl group Mn 2000 g/mol

A polyisobutyl benzene sulfonic acid is prepared by sulfonation of the polyisobutyl benzene from Example 3. The sulfonation is effected by reacting the polyisobutyl benzene with sulfur trioxide. The sulfonic acid group is mainly in para position to the polyisobutyl group, which can be determined by H-NMR.

### Example 7 -Polyisobutyl benzene sulfonate with Polyisobutyl group Mn 1000 g/mol

The polyisobutyl benzene sulfonic acid from Example 4 is neutralized with equimolar amounts of
- calcium hydroxide to yield polyisobutyl benzene sulfonate calcium salt.
- magnesium carbonate to yield polyisobutyl benzene sulfonate magnesium salt.

To make overbased sulfonates the polyisobutyl benzene sulfonic acid from Example 4 is is neutralized with a molar excess of
- calcium hydroxide to yield overbased polyisobutyl benzene sulfonate calcium salt.
- magnesium carbonate to yield overbased polyisobutyl benzene sulfonate magnesium salt.

### Example 8 -Polyisobutyl benzene sulfonate with polyisobutyl group Mn 1300 g/mol

The polyisobutyl benzene sulfonic acid from Example 5 is neutralized with equimolar amounts of
- calcium hydroxide to yield polyisobutyl benzene sulfonate calcium salt.
- magnesium carbonate to yield polyisobutyl benzene sulfonate magnesium salt.

To make overbased sulfonates the polyisobutyl benzene sulfonic acid from Example 5 is is neutralized with a molar excess of
- calcium hydroxide to yield overbased polyisobutyl benzene sulfonate calcium salt.
- magnesium carbonate to yield overbased polyisobutyl benzene sulfonate magnesium salt.

### Example 9 -Polyisobutyl benzene sulfonate with polyisobutyl group Mn 2000 g/mol

The polyisobutyl benzene sulfonic acid from Example 6 is neutralized with equimolar amounts of
- calcium hydroxide to yield polyisobutyl benzene sulfonate calcium salt.
- magnesium carbonate to yield polyisobutyl benzene sulfonate magnesium salt.

To make overbased sulfonates the polyisobutyl benzene sulfonic acid from Example 6 is is neutralized with a molar excess of
- calcium hydroxide to yield overbased polyisobutyl benzene sulfonate calcium salt.
- magnesium carbonate to yield overbased polyisobutyl benzene sulfonate magnesium salt.

## Claims

1. A method for preparing a polyisobutyl benzene sulfonate comprising the steps of
i) alkylation of benzene with a terminal unsaturated polyisobutene to produce a polyisobutyl benzene,
ii) sulfonation of the polyisobutyl benzene to produce a polyisobutyl benzene sulfonic acid, and
iii) neutralizing the polyisobutyl benzene sulfonic acid to produce a polyisobutyl benzene sulfonate,
where the terminal unsaturated polyisobutene comprises at least 50 mol% of terminal double bonds, based on the total number of polyisobutene macromolecules.

2. The method according to claim 1 where the terminal unsaturated polyisobutene is of the formula (b1), (b2), or mixtures thereof 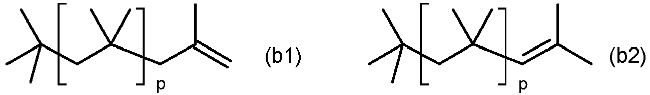 where p is in the range from 5 to 100, preferably from 8 to 80, and in particular from 10 to 50.

3. The method according to claim 1 or 2 where the terminal unsaturated polyisobutene comprises at least 60 mol% and in particular at least 80 mol%, based on the total number of polyisobutene macromolecules, of terminal double bonds.

4. The method according to any of the preceding claims where the polyisobutyl group has a molecular weight of 300 to 10.000 g/mol, preferably from 400 to 5000 g/mol, and in particular from 400 to 3000 g/mol.

5. The method according to any of the preceding claims where the polyisobutyl is a monovalent radical of the formula (a) 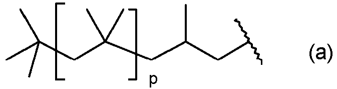 where p is in the range from 5 to 100, preferably from 8 to 80, and in particular from 10 to 50.

6. The method according to any of the preceding claims where the sulfonic acid group is in para position to the polyisobutyl group.

7. A polyisobutyl benzene sulfonate, where the polyisobutyl is a monovalent radical of the formula (a) 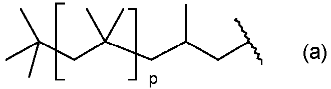 where p is in the range from 5 to 100, preferably from 8 to 80, and in particular from 10 to 50.

8. The polyisobutyl benzene sulfonate according to claim 7 where the polyisobutyl group has a molecular weight of 300 to 10.000 g/mol, preferably from 400 to 5000 g/mol, and in particular from 400 to 3000 g/mol.

9. The polyisobutyl benzene sulfonate according to claim 7 or 8, in form of a neutral or overbased alkali metal salt, earth alkali metal salt or ammonium salt.

10. The polyisobutyl benzene sulfonate according to any of claims 7 to 9 where the sulfonate group is in para position to the polyisobutyl group.

11. A polyisobutyl benzene sulfonic acid, where the polyisobutyl is a monovalent radical of the formula (a) 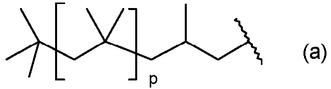 where p is in the range from 5 to 100, preferably from 8 to 80, and in particular from 10 to 50 .

12. The polyisobutyl benzene sulfonic acid according to claim 11 where the polyisobutyl group has a molecular weight of 300 to 10.000 g/mol, preferably from 400 to 5000 g/mol, and in particular from 400 to 3000 g/mol.

13. The polyisobutyl benzene sulfonic acid according to claim 11 or 12 having the general structure (II) 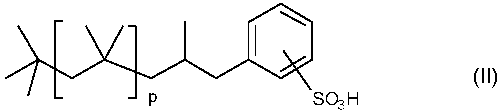 where p is in the range from 5 to 100.

14. The polyisobutyl benzene sulfonic acid according to claims 11 to 13 where the sulfonic acid group is in para position to the polyisobutyl group.

15. A polyisobutyl benzene, where the polyisobutyl is a monovalent radical of the formula (a) 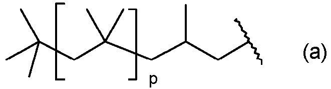 where p is in the range from 5 to 100, preferably from 8 to 80, and in particular from 10 to 50.

16. The polyisobutyl benzene according to claim 15 where the polyisobutyl group has a molecular weight of 300 to 10.000 g/mol, preferably from 400 to 5000 g/mol, and in particular from 400 to 3000 g/mol.

17. A lubricant, preferably a lubricating liquid, a lubricating oil or a lubricating grease, comprising the polyisobutyl benzene sulfonate as defined in any of claims 7 to 10 or obtainable according to the method as defined in any of claims 1 to 6.

18. A fuel comprising the polyisobutyl benzene sulfonate as defined in any of claims 7 to 10 or obtainable according to the method as defined in any of claims 1 to 6.

19. A use of the polyisobutyl benzene sulfonate as defined in any of claims 7 to 10 or obtainable according to the method as defined in any of claims 1 to 6 as lubricant additive or fuel additive.

## Patentansprüche

1. Eine Methode zur Herstellung eines Polyisobutylbenzolsulfonats, bestehend aus den Schritten von
i) Alkylierung von Benzol mit einem terminalen ungesättigten Polyisobuten zur Erzeugung eines Polyisobutylbenzols,
ii) Sulfonation des Polyisobutylbenzols zur Bildung einer Polyisobutylbenzol-Sulfonsäure, und
iii) indem die Polyisobutylbenzol-Sulfonsäure neutralisiert wird, um ein Polyisobutylbenzolsulfonat zu erzeugen,
wobei das terminal ungesättigte Polyisobuten mindestens 50 mol % der terminalen Doppelbindungen ausmacht, basierend auf der Gesamtzahl der Polyisobuten-Makromoleküle.

2. Die Methode gemäß Anspruch 1, bei der das terminale ungesättigte Polyisobuten aus der Formel (b1), (b2) oder deren Mischungen stammt 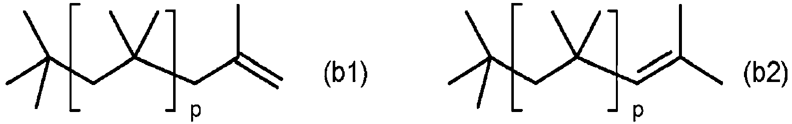 wobei p im Bereich von 5 bis 100 liegt, vorzugsweise von 8 bis 80, insbesondere von 10 bis 50.

3. Die Methode nach Behauptung 1 oder 2, bei der das terminale ungesättigte Polyisobuten mindestens 60 Mol % und insbesondere mindestens 80 mol % enthält, basierend auf der Gesamtzahl der Polyisobuten-Makromoleküle, mit terminalen Doppelbindungen.

4. Die Methode entspricht einer der vorangegangenen Behauptungen, wobei die Polyisobutylgruppe ein Molekulargewicht von 300 bis 10,000 g/mol hat, vorzugsweise von 400 bis 5000 g/mol, insbesondere von 400 bis 3000 g/mol.

5. Die Methode gemäß einer der vorangegangenen Behauptungen, bei denen das Polyisobutyl ein monovalentes Radikal der Formel (a) ist 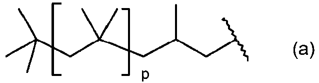 wobei p im Bereich von 5 bis 100 liegt, vorzugsweise von 8 bis 80, insbesondere von 10 bis 50.

6. Die Methode entspricht einer der vorangegangenen Behauptungen, bei der die Sulfonsäuregruppe in para Position zur Polyisobutylgruppe liegt.

7. Ein Polyisobutyl-Benzolsulfonat, wobei das Polyisobutyl ein monovalentes Radikal der Formel (a) ist 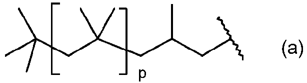 wobei p im Bereich von 5 bis 100 liegt, vorzugsweise von 8 bis 80, insbesondere von 10 bis 50.

8. Das Polyisobutylbenzolsulfonat laut Anspruch 7, wobei die Polyisobutylgruppe ein Molekulargewicht von 300 bis 10.000 g/mol hat, vorzugsweise von 400 bis 5000 g/mol, insbesondere von 400 bis 3000 g/mol.

9. Das Polyisobutyl-Benzolsulfonat nach Behauptung 7 oder 8 ist in Form eines neutralen oder überbasierten Alkalimetallsalzes, Erdalkalisalz oder Ammoniumsalz.

10. Das Polyisobutyl-Benzolsulfonat gemäß einem der Ansprüche 7 bis 9, bei denen die Sulfonatgruppe para zur Polyisobutylgruppe steht.

11. Eine Polyisobutylbenzol-Sulfonsäure, bei der das Polyisobutyl ein monovalentes Radikal der Formel (a) ist 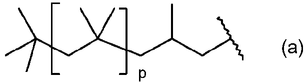 wobei p im Bereich von 5 bis 100 liegt, vorzugsweise von 8 bis 80, insbesondere von 10 bis 50.

12. Die Polyisobutylbenzol-Sulfonsäure laut Anspruch 11, wobei die Polyisobutylgruppe ein Molekulargewicht von 300 bis 10.000 g/mol hat, vorzugsweise zwischen 400 und 5000 g/mol, insbesondere zwischen 400 und 3000 g/mol.

13. Die Polyisobutylbenzol-Sulfonsäure hat laut Behauptung 11 oder 12 die allgemeine Struktur (II) 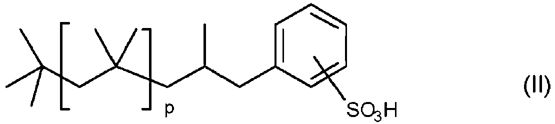 wobei p im Bereich von 5 bis 100 liegt.

14. Die Polyisobutylbenzol-Sulfonsäure laut Angaben 11 bis 13, wobei die Sulfonsäuregruppe para zur Polyisobutylgruppe liegt.

15. Ein Polyisobutylbenzol, wobei das Polyisobutyl ein monovalentes Radikal der Formel (a) ist 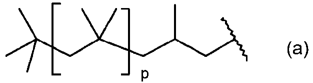 wobei p im Bereich von 5 bis 100 liegt, vorzugsweise von 8 bis 80, insbesondere von 10 bis 50.

16. Das Polyisobutylbenzol laut Behauptung 15, wobei die Polyisobutylgruppe ein Molekulargewicht von 300 bis 10,000 g/mol hat, vorzugsweise zwischen 400 und 5000 g/mol, insbesondere zwischen 400 und 3000 g/mol.

17. Ein Schmiermittel, vorzugsweise eine Schmierflüssigkeit, ein Schmieröl oder ein Schmierfett, das das Polyisobutylbenzolsulfonat gemäß einer der Ansprüche 7 bis 10 umfasst oder nach der in jedem der Ansprüche 1 bis 6 definierten Methode erhältlich ist.

18. Ein Brennstoff, der das Polyisobutylbenzolsulfonat wie in einem der Ansprüche 7 bis 10 definiert oder nach der in jedem der Ansprüche 1 bis 6 definierten Methode erhältlich ist.

19. Eine Verwendung des Polyisobutylbenzolsulfonats, wie es in einem der Ansprüche 7 bis 10 definiert ist oder nach der Methode gemäß den Forderungen 1 bis 6 als Schmierstoffzusatz oder Kraftstoffzusatz erhältlich ist.

## Revendications

1. Une méthode pour préparer un sulfonate de polyisobutyle benzène comprenant les étapes de
i) alkylation du benzène avec un polyisobutène insaturé terminal pour produire un polyisobutyle benzène,
ii) la sulfonation du polyisobutyle benzène pour produire un acide sulfonique polyisobutyle benzène, et
iii) neutralisant l'acide sulfonique de polyisobutyle benzène pour produire un sulfonate de polyisobutyle benzène,
où le polyisobutène insaturé terminal comprend au moins 50 % de liaisons doubles terminales, basé sur le nombre total de macromolécules de polyisobutène.

2. La méthode selon l'affirmation 1 où le polyisobutène insaturé terminal appartient à la formule (b1), (b2), ou à ses mélanges 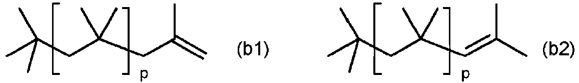 où p est dans la plage de 5 à 100, de préférence de 8 à 80, et en particulier de 10 à 50.

3. La méthode, selon la revendication 1 ou 2, où le polyisobutène insaturé terminal comprend au moins 60 mol % et en particulier au moins 80 mol %, basé sur le nombre total de macromolécules de polyisobutène de double liaison terminale.

4. La méthode, selon l'une des affirmations précédentes, où le groupe polyisobutyle a un poids moléculaire de 300 à 10 000 g/mol, de préférence entre 400 et 5000 g/mol, et en particulier entre 400 et 3000 g/mol.

5. La méthode selon l'une des affirmations précédentes où le polyisobutyle est un radical monovalent de la formule (a) 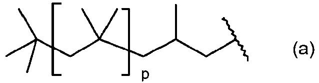 où p est dans la plage de 5 à 100, de préférence de 8 à 80, et en particulier de 10 à 50.

6. La méthode selon l'une des affirmations précédentes où le groupe acide sulfonique est en position para par rapport au groupe polyisobutyle.

7. Un sulfonate de polyisobutyle benzène, où le polyisobutyle est un radical monovalent de la formule (a) 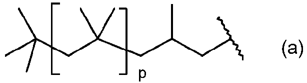 où p est dans la plage de 5 à 100, de préférence de 8 à 80, et en particulier de 10 à 50.

8. Le sulfonate de benzène polyisobutyle selon la revendication 7 où le groupe polyisobutyle a un poids moléculaire de 300 à 10 000 g/mol, de préférence entre 400 et 5000 g/mol, et en particulier entre 400 et 3000 g/mol.

9. Le sulfonate de polyisobutyle benzène selon la revendication 7 ou 8, sous forme de sel métallique alcalin neutre ou surchargé, sel métallique alcalin terrestre ou sel d'ammonium.

10. Le polyisobutyle benzène sulfonate selon l'une des revendications 7 à 9, où le groupe sulfonate est en position para par rapport au groupe polyisobutyle.

11. Un acide sulfonique polyisobutyle benzène, où le polyisobutyle est un radical monovalent de la formule (a) 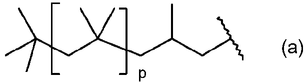 où p est dans la plage de 5 à 100, de préférence de 8 à 80, et en particulier de 10 à 50.

12. L'acide polyisobutyle benzène sulfonique selon la revendication 11 où le groupe polyisobutyle a un poids moléculaire de 300 à 10 000 g/mol, de préférence entre 400 et 5000 g/mol, et en particulier entre 400 et 3000 g/mol.

13. L'acide sulfonique polyisobutyle benzène selon la revendication 11 ou 12 a la structure générale (II) 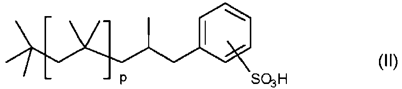 où p est dans l'intervalle de 5 à 100.

14. L'acide sulfonique de polyisobutyle benzène selon les revendications 11 à 13 où le groupe acide sulfonique est en position para par rapport au groupe polyisobutyle.

15. Un polyisobutyle benzène, où le polyisobutyle est un radical monovalent de la formule (a) 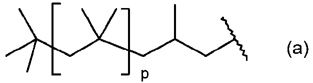 où p est dans la plage de 5 à 100, de préférence de 8 à 80, et en particulier de 10 à 50.

16. Le polyisobutyle benzène selon la revendication 15 où le groupe polyisobutyle a un poids moléculaire de 300 à 10 000 g/mol, de préférence entre 400 et 5000 g/mol, et en particulier entre 400 et 3000 g/mol.

17. Un lubrifiant, de préférence un liquide lubrifiant, une huile lubrifiante ou une graisse lubrifiante, composé du sulfonate de polyisobutyle benzène tel que défini dans l'une des revendications 7 à 10 ou disponible selon la méthode définie dans l'une des revendications 1 à 6.

18. Un combustible composé du sulfonate de polyisobutyle benzène tel que défini dans l'une des revendications 7 à 10 ou disponible selon la méthode définie dans l'une des revendications 1 à 6.

19. Une utilisation du sulfonate de polyisobutyle benzène telle que définie dans l'une des revendications 7 à 10 ou obtenue selon la méthode telle que définie dans l'une des revendications 1 à 6, comme additif lubrifiant ou additif carburant.
